Europäisches Patentamt
European Patent Office
Office européen des brevets

(11) Publication number: 0 222 533 A1

# EUROPEAN PATENT APPLICATION

(21) Application number: 86308174.1

(22) Date of filing: 21.10.86

(51) Int. Cl.4: A61K 31/165 , A61K 31/40 , C07D 295/12

(30) Priority: 25.10.85 US 791311

(43) Date of publication of application: 20.05.87 Bulletin 87/21

(84) Designated Contracting States: AT ES GR

(71) Applicant: THE UPJOHN COMPANY
301 Henrietta Street
Kalamazoo, Michigan 49001(US)

(72) Inventor: Szmuszkovicz, Jacob
The Upjohn Company 301 Henrietta Street
Kalamazoo Michigan 49001(US)
Inventor: von Voigtlander, Philip F.
The Upjohn Company 301 Henrietta Street
Kalamazoo Michigan 49001(US)

(74) Representative: Perry, Robert Edward et al
GILL JENNINGS & EVERY 53-64 Chancery Lane
London WC2A 1HN(GB)

(54) **Cis-N-[(2-aminocycloaliphatic)benzene acetamide and -benzamide anticonvulsants.**

(57) Certain cis-N-(2-amino-cycloaliphatic)benzeneacetamide and -benzamides of the formula

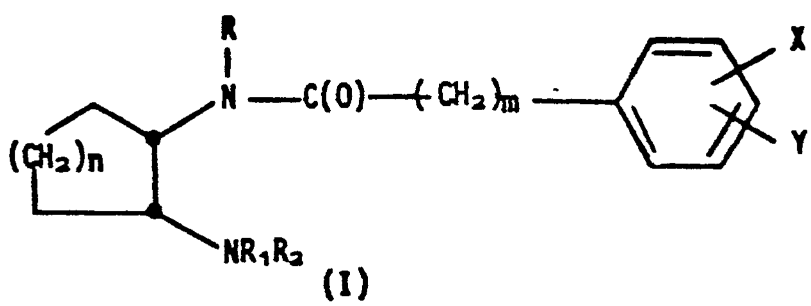

(I)

(I)

where m is 0 or 1, n is 1 or 2, R is hydrogen or alkyl, $R_1$ and $R_2$ are separately alkyl, or, taken together with the nitrogen denote a pyrrolidine ring, and X and Y are hydrogen, fluorine, chlorine or bromine or one of X and Y is trifluoromethyl, e.g., cis-3,4-dichloro-N-methyl-N-[2-(1-pyrrolidinyl)cyclohexyl]benzamide and salts thereof, have been found to have CNS seizure blocking or preventing drug action, e.g., anti-convulsant drug properties with little or no analgesic properties. Some of these compounds are new.

EP 0 222 533 A1

# CIS-N-[(2-AMINOCYCLOALIPHATIC)BENZENE ACETAMIDE AND -BENZAMIDE ANTICONVULSANTS

## INTRODUCTION

This invention relates to the use of certain N-(2-amino cycloaliphatic)benzeneacetamide and -benzamide compounds as Central Nervous System (CNS) anti-seizure, e.g., anti-convulsant, drugs in valuable warm-blooded animals, including humans.

## BACKGROUND OF THE INVENTION

Szmuszkovicz U.S. Patent No. 4,098,904 discloses some N-(2-aminocycloaliphatic)benzamide compounds, e.g., N-methyl-N-[2-(1-pyrrolidinyl)cyclohexyl]-3,4-dichlorobenzamide, and N-methyl-N-[2-(N',N'-dimethylamino)cyclohexyl]-4-bromobenzamide, and salts and hydrates thereof as analgesic (pain reducing) drug compounds.

Szmuszkovicz U.S. Patent No. 4,145,435 discloses some 2-aminocycloaliphatic alkanoyl amide compounds, e.g., N-methyl-N-[2-(N',N'-dimethylamino)-cyclohexyl]-2-(4-bromophenyl)acetamide and trans-2-(3,4-dichlorophenyl-N-methyl-N-[2-(1-pyrrolidinyl)cyclohexyl]acetamide, and their pharmaceutically acceptable salts as analgesic compounds.

In contrast to the disclosure in those Szmuszkovicz '904 and '435 patents, the compounds of this invention have little or no significant analgesic activity ($ED_{50}$ >50), but have been found to possess significant CNS seizure preventing or blocking activity in standard laboratory animal tests.

In Neuroscience Abstracts, 10, page 408 (October, 1984), F.C.Tortella et al report the Seizure-specific, Dose-and Time-Dependant Anti-convulsant Profile of Upjohn Compound, Trans-(±)-3,4-dichloro-N-methyl-N-[2-(1-pyrrolidinyl)cyclohexyl]benzeneacetamide by an electroshock test method. This compound is described and claimed in the above Szmuszkovicz '435 patent; however, as indicated hereinabove, this compound is an analgesically active compound.

Recent pharmacology articles that may be of interest as background are:

J. Med. Chem. , 1984, 27, 779-782, "Anticonvulsant ... 4-Aminobenzamides" by C. Randall Clark et al,

Science, 226, 16 November 1984, pp. 850-852, "Blockade ... Against Ischemic Damage in the Brain" by R. P. Simon et al, and

Neuropharmacology, 23, No. 3, pp. 367-371 (1984), "The Anti-convulsant ... Electroshock Seizures in Rats" by E. F. Berman et al, although these references are not conceded to be prior art to the invention described and claimed herein.

## OBJECTS OF THE INVENTION

It is an object of this invention to provide a new use for some N-(2-aminocycloaliphatic)-benzeneacetamide and -benzamide compounds, some of which are new, which have been found to have significant CNS seizure preventing and blocking activity, while having only minimal, if any, analgesic activity.

It is a further object of this invention to provide a method for preventing or blocking CNS seizures in warm-blooded valuable animal patients, including humans with certain N-(2-aminocycloaliphatic)-benzeneacetamides and -benzamides.

Another object of the invention is to provide pharmaceutical compositions useful in dosage unit form for administration to valuable warm-blooded animal patients, including humans, for prevention or blocking of CNS seizures in such patients.

Other objects, aspects and advantages of the invention will be apparent from reading the remaining specification and the claims which follow.

## SUMMARY OF THE INVENTION

Briefly, this invention provides the new use of a group of cis-N-[(2-aminocycloaliphatic)-benzeneacetamide and -benzamide compounds, of general formula I, and their pharmaceutically acceptable salts, which compounds have been discovered to have significant CNS seizure-preventing activity, while also showing only minimal or non-significant, analgesic activity, in standard laboratory animal tests.

This invention also includes pharmaceutical compositions containing these compounds and a method for treating warm-blooded animal patients, including humans, with these compositions to prevent or block CNS seizures of various types in such patients.

## DETAILED DESCRIPTION OF THE INVENTION

The group of compounds described herein have been found to be effective anti-convulsant drug agents in standard animal tests. These compounds have been shown to be effective antagonists of electroshock seizures in mice. They will be useful in the treatment of grand mal and other seizure disorders in man as well as in commercially important and pet animals.

These compounds are active in the CNS electroshock animal test but they did not at similar dosage ranges block chemically induced (pentylenetetrazol or bicucullin) seizures in the test animals suggesting that these compounds have a CNS anti-seizure utility similar to that of the known drug phenytoin (THE MERCK INDEX, 10th. Edition, page 1054, item 7204). Phenytoin is useful in the treatment of grand mal, focal and psychomotor seizures (see Goodman and Gilman, The Pharmacological Basis of Therapeutics). However, phenytoin and structurally related anti-convulsant drugs are known to cause a number of side effects including hyperplasia of the gums, cerebellar degeneration, gastric distress and serious skin rashes.

In contrast, the compounds of this invention, while being anti-seizure drugs, e.g., anti-convulsant drugs, at reasonable dosages, have little or no significant analgesic activity, and it is also hoped that these cis-compounds will not show some of the other above-listed side effects as well. The anti-convulsant potency of these cis-amino-amide compounds in standard laboratory animal tests indicate that these compounds will be useful for preventing or treating CNS seizure disorders in warm-blooded animals such as cats, dogs, horses, as well as humans at dosage ranges of approximately 0.1 to 100 mg./kg. of body weight/day via the oral or parenteral routes until the seizure threat or attack subsides.

The compounds of this invention, of formula I in the GENERAL FORMULAS hereinbelow, are cis-N-[(2-aminocycloaliphatic)benzeneacetamide and -benzamide compounds where the two large filled circle positions in the ring are intended to depict the cis orientation of the two nitrogen groups relative to each other and the ring,

n is 1 or 2, to indicate a cyclopentyl or cyclohexyl ring;

m is 0 or 1, to indicate that the compounds are benzeneacetamides (with the $-CH_2-$) or benzamides (without the $-CH_2-$),

R is hydrogen or $C_1$ to $C_3$-alkyl, e.g., methyl, ethyl, isopropyl or n-propyl, preferably methyl, when R is $C_1$ to $C_3$-alkyl,

$R_1$ and $R_2$ taken separately, are independently $C_1$ to $C_3$-alkyl, as defined above, or

$R_1$ and $R_2$ can be taken together with the nitrogen to which they are bonded to complete a pyrrolidine ring,

X and Y are each hydrogen, a halogen having an atomic number of from 9 to 35, that is, fluorine, chlorine or bromine, trifluoromethyl, preferably chlorine, in the 3-and the 4-position or bromine in the 4-position, or trifluoromethyl in the 4-position, and when

X and Y are each hydrogen, R is preferably hydrogen, m is 0, n is 1 or 2, and $R_1$ and $R_2$ are each $C_1$ to $C_3$-alkyl, preferably methyl, or a pharmaceutically acceptable salt thereof.

Acid addition salts of these amino-amides (I) can be prepared by reacting a Formula I free base with a stoichiometric amount of an acid, such as hydrogen chloride, hydrogen bromide, hydrogen iodide, sulfuric acid, phosphoric acid, acetic acid, lactic acid, citric acid, succinic acid, benzoic acid, salicyclic acid, pamoic acid, cyclohexanesulfamic acid, methanesulfonic, naphthalenesulfonic, p-toluenesulfonic, maleic, fumaric, oxalic acids and the like. The reaction can be carried out in aqueous or organic liquid solvent non-aqueous media such as diethyl ether, ethyl acetate, and the like. Non-aqueous media are preferred.

On occasion the compounds (I) or their acid addition salts in their crystalline state are isolated as solvates, e.g., with a discrete quantity of solvent, e.g., water, methanol, and the like, associated physically, and thus not affecting the chemical entity per se.

This invention also relates to compositions containing a Formula I compound as an active ingredient in a pharmaceutical carrier. The compositions are useful in pharmaceutical dosage unit forms of the Formula I compounds for systemic administration (oral, rectal and parenteral (including intravenous, intramuscular and intra-arterial) administration form) for treating warm-blooded animal patients, cats, dogs, horses and other commercially valuable animals, and human patients suspected of being susceptible to or to stop CNS seizures, such as convulsions, grand mal, petit mal and other seizure disorders. The term "dosage unit form" as used in this specification and in the claims refers to physically discrete units suitable as unitary dosages for mammalian subjects, each unit containing a predetermined quantity of the essential active ingredient compound of this invention calculated to produce the desired effect in combination with the required pharmaceutical means which adapt the said ingredient for topical or systemic administration. The specifications for the novel dosage unit forms of this invention are indicated by and directly dependent on the physical characteristics of the essential active ingredient and the particular effect to be achieved in view of the limitations inherent in the art of compounding such an essential material for beneficial effects in humans and animals. Examples of suitable dosage unit forms in accordance with this invention are tablets, capsules, orally administered liquid preparations in suitable liquid vehicles, sterile preparations in suitable liquid vehicles for intramuscular and intravenous administration, suppositories, and sterile dry preparations for the extemporaneous preparation of sterile injectable preparations in a suitable liquid vehicle. Suitable solid diluents or carriers for the solid oral pharmaceutical dosage unit forms are selected from the group consisting of lipids, carbohydrates, proteins and mineral solids, for example, starch, sucrose, lactose, kaolin, dicalcium phosphate, gelatin, acacia, corn syrup starch, talc and the like. Capsules both hard and soft are filled with composition of these amino amide ingredients in combination with suitable diluents and excipients, for example, edible oils, talc, calcium carbonate and the like and also calcium stearate. Liquid preparations for oral administration are prepared in water or aqueous vehicles which advantageously contain suspending agents, for example, methylcellulose, acacia, polyvinylpyrrolidone, polyvinyl alcohol and the like. In the case of injectable forms, the injectable formulation must be sterile and must be fluid to the extent that easy syringeability exists. Such preparations must be stable under the conditions of manufacture and storage, and ordinarily contain in addition to the basic solvent or suspending liquid, preservatives in the nature of bacteriostatic and fungistatic agents, for example, parabens, chlorobutanol, benzyl alcohol, phenol, thimerosal, and the like. In many cases it is preferably to include osmotically active agents, for example, sugars or sodium chloride in isotonic concentrations. Carriers and vehicles include vegetable oils, ethanol, polyols, for example, glycerol, propylene glycol, liquid polyethylene glycol, and the like. Any solid preparations for subsequent extremporaneous preparation of sterile injectable preparations are sterilized, preferably by exposure to a sterilizing gas, for example, ethylene oxide. The aforesaid carriers, vehicles, diluents, excipients, preservatives, isotonic agents and the like constitute the pharmaceutical means which adapt the preparations for systemic administration.

The pharmaceutical dosage unit forms are prepared in accordance with the preceding general description to provide from about 1.0 to about 700 mg. of the essential active ingredient per dosage unit form, which as aforesaid may be in the form of a solid, semi-solid, topical, oral, or rectal preparation, a liquid oral preparation, an injectable preparation, including liquid preparations and solid dry preparations for extemporaneous reconstitution to a liquid injectable preparation. The amount of the essential active ingredient provided in the pharmaceutical dosage unit forms is that amount sufficient to obtain a reduction in the CNS seizure effects and a return to more normal CNS stability, or to prevent the occurrence of CNS seizures in a patient who is suspected to be subject to such seizure. Expressed otherwise an amount of the essential active ingredient is provided to a recipient within a range from about 0.1 mg. per kg. to about 100 mg. per kg. of body weight of the recipient. Preferred dosages for most applications are 1.0 to 10.0 mg. per kg. of body weight.

The useful pharmaceutical dosage unit forms of these compounds in pharmaceutical formulations is preferably adapted for systemic administration to obtain anti-convulsant effects comprising an effective, non-toxic amount of a compound according to Formula I or as its pharmacologically acceptable salt. Further the invention relates to methods of obtaining such CNS anti-seizure effects in mammals, for example, humans and valuable warm-blooded animals such as dogs, cats, horses and other commercially valuable animals, by administering systemically to the mammals the aforesaid pharmaceutical dosage unit forms supplying an effective, nontoxic amount for anticonvulsant effects.

These specific cis-amino-amide compounds have an advantage, to a greater or lesser extent, depending upon the particular compound of having significant CNS antiseizure properties, while having little or no significant analgesic activity (analgesic $ED_{50}$ >50 in the HCl writhing test). This combination of properties should prove beneficial and advantageous where the doctor would prefer to treat the patient for the single CNS seizure disorder without need to worry about any significant analgesic side effects.

The cis-amino-amide compounds involved in this invention can be made by procedures described in the Szmuszkovicz U.S. Patents 4,098,904 and 4,145,435, supra, using the cis-1,2-diamines referred to in those two patents, and the appropriate acylating form of the selected benzeneacetic acid, or -acetyl halide, or the selected benzoic acid, benzoyl halide, as described generally therein. Described hereinbelow are procedures used to prepare the compounds described and exemplified herein. SCHEME I and II attached hereto, show a typical procedure for preparing the compounds involved in this invention.

Scheme I outlines in general chemical symbol form a process for preparing cis-diamine starting materials, which can be used to make the cis-2-amino-cycloaliphatic-benzeneacetamide and -benzamide compounds, which are used according to the invention described herein.

In general, the alkyl cycloalkanone carboxylate ester starting material (II), which can be, e.g., the pure methyl, ethyl or propyl esters or the commercially available mixed esters, and the selected $HNR_1R_2$ amine, e.g., pyrrolidine, dimethylamine, diethylamine, di-n-propylamine, di-ispropylamine, or a mixed amine such as N-methyl-N-ethylamine, are mixed and reacted in a selected solvent such as benzene, toluene, heptane, or the like, while heating under reflux conditions to remove water by-product from the reaction mixture to form the respective alkyl 2-aminocycloalk-1-enylcarboxylate ester (III). Alternatively, the alkyl 2-aminocycloalk-1-enylcarboxylate ester (III) can be prepared from a 1-cycloalkenylamine (IV) and a carboxylation agent.

The resulting 2-aminocycloalk-1-enylcarboxylate ester (III) can then be reduced, e.g., by hydrogenating the reaction mixture containing the ester (III) in the presence of a hydrogenation catalyst such as platinum oxide to form the saturated cis 2-aminocycloaliphatic ester (V).

The cis-saturated ring amino-ester (V) can then be treated with hydrazine in the presence of an appropriate solvent, such as a $C_1$ to $C_4$-alkanol, under reflux conditions to form the cis-amino-hydrazide (VI). The hydrazide (VI) can be subjected to a nitrosation-Curtius rearrangement reaction to form the cis-2-aminocycloalkyl primary amine (VII) which can be used directly to make end product compounds of structure I herein where R is hydrogen.

For compounds where R is to be a $C_1$ to $C_3$-alkyl, the cis-2-aminocycloalkylamine VII is further acylated with an alkyl formate (to form an R equals methyl compound, or with an acetyl halide or propionyl halide, e.g., acetyl chloride or propionyl chloride, in the presence of a tertiary amine hydrogen halide scavenger to form the cis-N-(2-aminocycloalkyl) $C_1$ to $C_3$-alkanoylamide (VIII), where $R_3$ is hydrogen or $C_1$ to $C_2$-alkyl. This resulting amino-amide (VIII) can then be subjected to carbonyl group reduction, e.g., with lithium aluminum hydride, to convert the $R_3$-C(O)-group on the nitrogen in the 1-position of the cycloalkyl ring to the defined R equals $C_1$ to $C_3$-alkyl group, and to form cis-diamine (IX).

Scheme II describes in chemical symbol form an amine nitrogen acylation process that can be used to make the cis N-[2-(aminocycloaliphatic)-N-benzeneacetamide (m = 1) and -benzamide (m = 0) compounds which can then be recovered from their reaction mixtures, and used according to this invention or converted to appropriate acid addition salt form for recovery from its reaction mixture, and then re-formed, if desired or necessary, into an appropriate, pharmaceutically acceptable acid addition salt form for compounding into appropriate marketing and dosage unit forms.

Here, in Scheme II the cis-cycloaliphatic diamine (IX) can be acylated, by known procedures, e.g., by reaction with a benzeneacetyl halide or benzoyl halide (X), in the presence of a tertiary amine hydrogen halide scavenger, such as triethylamine or N-N-dimethylaniline to form the N-[2-(aminocycloalkyl)-N-(H-or alkyl)benzeneacetamine or-benzamide. These latter compounds can then be processed as indicated herein to put the compounds in pharmaceutically acceptable useful form.

Preparation CIS-2-PYRROLIDINYLCYCLOHEXYLAMINE

This preparation exemplifies how cis-1,2-cyclohexanediamines were prepared, for use in preparing the cis-amino-amide compounds which are involved in the invention described and claimed herein.

A. Ethyl cis-2-pyrrolidinylcyclohexanecarboxylate

A solution of 164.4 g (1.0 mole) ethyl 2-cyclohexanone carboxylate [also named ethyl 2-oxocyclohexanoate], which also contained about 40 percent of the corresponding methyl ester, and 71.1 g. - (1.0 mole) of pyrrolidine in 700 ml. of benzene was refluxed overnight with azeotropic distillation of water therefrom (18 ml. $H_2O$ collected) to form the ethyl and methyl 2-(1-pyrrolidinyl)-1-cyclohexen-1-ylcarboxylate mixed ester. Then 10 g. of platinum oxide was added to this resulting reaction mixture, and the

mixture was hydrogenated at 30 psig. until uptake of hydrogen ceased. About 0.95 mole of hydrogen was consumed. The resulting hydrogenated reaction mixture contained the cis-ethyl and methyl mixed 2-(1-pyrrolidinyl)-cyclohexanecarboxylate esters. This reaction mixture was filtered through a filter aid (CELITE ®) to remove the platinum catalyst residue. The filtered liquid was then extracted with 200 ml. of 6N hydrochloric acid. The resulting aqueous layer was washed with ether, made basic with 15 percent sodium hydroxide and again extracted with ether. The original organic layer and the ether wash were combined as one organic layer and washed with saturated sodium chloride in water solution, dried ($MgSO_4$) and evaporated to remove solvents. Distillation of the residue gave the cis-2-(1-pyrrolidinyl)-cyclohexanecarboxylate, mixed methyl and ethyl esters, 143.5 g. (70 percent yield, b.p. 95°-105°C./0.5 mm. (Hg.).

This cis-amino-cyclohexanecarboxylate mixed ester material was used in the next step of the process without further purification.

B. Cis-2-Pyrrolidinylcyclohexanecarboxhydrazide, and its hydrochloride.

A solution of 143.5 g. (0.65 mole) of the named amino-mixed ester material from Part A hereinabove and 162.5 g. (3.25 moles) of hydrazine hydrate in 1300 ml. of absolute ethanol was stirred at room temperature for 24 hours, and then refluxed for 72 hours to form the 2-(1-pyrrolidinyl)-cyclohexanecarboxhydrazide. The solvent was largely removed by evaporation. The residue was diluted with 1500 ml. of methylene chloride, washed twice with 150 ml. portions of saturated aqueous sodium chloride solution, dried ($MgSO_4$) and evaporated to remove most of the methylene chloride.

The 2-(1-pyrrolidinyl)cyclohexanecarboxhydrazide hydrochloride salt was prepared by treatment of this hydrazide residue with hydrogen chloride in ether solution and was recrystallized from a methanol/ether solvent mixture to give the cis-2-(1-pyrrolidinyl)cyclohexanecarboxhydrazide hydrochloride, 126.8 g., (69 percent yield), m.p. 222°-224°C. The UV, mass spectrum, IR and NMR spectra were consistent with this named compound.

Anal. Calcd. for $C_{11}H_{21}N_3O \bullet 2HCl$:
% Calcd: C, 46.48; H, 8.16; N, 14.78; Cl 24.95
Found: C, 45.49; H, 8.22; N, 15.20; Cl 25.50

C. Cis-2-(1-Pyrrolidinyl)cyclohexaneamine

To a solution of 53.4 g. (0.188 mole) of the 2-(1-pyrrolidinyl)cyclohexanecarboxhydrazide hydrochloride in 100 ml. of water, prepared as in Part B hereinabove with ice cooling there was added 14.3 g. (0.207 mole) of sodium nitrite in 25 ml. of water over 30 minutes, while stirring. After 30 minutes 30 ml. of 12N hydrochloric acid was added to the reaction mixture and the solution was slowly heated to 70°C. on a water bath. When evolution of nitrogen by-product had nearly ceased, the solution was heated on a steam bath for 1 hour. The resulting solution was cooled, made basic with 15 percent sodium hydroxide solution, and extracted with ether. The ether extract was dried ($MgSO_4$) and evaporated to remove solvent. Distillation of the residue gave 25.7 g. (81 percent yield) of the diamine, cis-2-(1-pyrrolidinyl)cyclohexaneamine, b.p. 120°-123°C./14 mm. (Hg.). The UV, IR, NMR and Mass spectra were consistent with this named compound.

Anal. Calcd. for $C_{10}H_{20}N_2$:
% Calcd.: C, 71.37; H, 11.98; N, 16.65
Found: C, 71.05; H, 11.91; N, 16.49

D. N-Methyl-Cis-2-(1-Pyrrolidinyl)cyclohexylamine

A solution of 20.0 g. (0.119 mole) of the cis-2-(1-pyrrolidinyl)-cyclohexaneamine, from Part C hereinabove in 250 ml. of ethyl formate solvent was refluxed overnight. The solution was evaporated to remove solvent and leave a crude amide intermediate as a colorless oil product. The crude product was dissolved in 100 ml. of tetrahydrofuran (THF) and added dropwise in one hour to a suspension of 20.0 g. of lithium aluminum hydride in 1000 ml. of ether. The mixture was refluxed for 6 hours and then kept overnight at room temperature. The mixture was cooled in ice and excess lithium aluminum hydride was decomposed by adding 20 ml. of water, 20 ml. of 15 percent sodium hydroxide aqueous solution and 60 ml. of water. The mixture was filtered and the filtered by-product aluminum salt solids were washed with ether. The

filtrate and ether washings were dried ($MgSO_4$) and evaporated to remove solvents. Distillation of the residue gave 16.5 g. (76 percent yield) of the cis-N-methyl-N-[2-(1-pyrrolidinyl)cyclohexaneamine, b.p. 120°-122°C./14 mm. (Hg.). The UV, IR, NMR and Mass Spectra were consistent with this named compound.

Anal. Calcd. for $C_{11}H_{22}N_2$:
% Calcd: C, 72,46; H, 12.17; N, 15.37
Found : C, 72.64; H, 12.73; N, 15.42

Example 1 Cis-N-[2-(N' ,N'-dimethylamino)cyclohexyl]benzamide

To a mixture of 1.42 g. (0.01 mole) of cis-[2-(dimethylamino)]cyclohexylamine (prepared as described in U.S. Patent No. 4,098,904 starting at column 21, line 67) and 1.01 g. (0.01 mole) of triethylamine in 100 ml. of diethyl-ether there was added 1.41 g. (0.01 mole) of benzoyl chloride in 25 ml. of ether over 30 minutes with ice cooling of the reaction vessel. The reaction mixture was stirred overnight at room temperature. Then, 100 ml. of a saturated sodium bicarbonate in water solution was added to the resulting reaction mixture. The aqueous layer was separated from the organic liquid layer. The organic liquid layer was then washed with brine (saturated aqueous sodium chloride), separated from the aqueous brine layers and dried using anhydrous magnesium sulfate and then evaporated to remove solvent and to leave a yellow oil residue, which residue became solid on standing, and which weighed 2.43 g. (99 percent, crude yield) of the cis-N-[2-(dimethylamino)-cyclohexyl]-N-benzamide, m.p. 73°-76°C.

The hydrochloride salt of this cis-N-[2-(dimethylamino)cyclohexyl]benzamide was made by dissolving this amino-amide compound in excess hydrogen chloride in ether solution, and then evaporating solvent therefrom to crystallize the hydrochloride salt therefrom. This amino-amide hydrochloride salt was recrystallized from a mixture of 10 ml. of methanol and about 40 ml. of ether to obtain 2.13 g. (75 percent yield) of the cis-N-[2-(dimethylamino)cyclohexyl]benzamide hydrochloride, m.p. 247°-248°C.

The elemental analysis for this amino-amide salt was:
Anal. Calcd. for $C_{15}H_{22}N_2O \bullet HCl$(282.81)
% Calcd.: C, 63.70; H, 8.20; N, 9.91; Cl, 12.54
Found: C, 63.90; H, 8.53; N, 9.92; Cl, 12.69

The Nuclear Magnetic Resonance (NMR) Spectrum ($D_2O$) was consistent for this named product. The Infrared (IR) spectrum was also consistent. The Ultraviolet (UV) light spectrum showed (ethyl alcohol) end absorption 227 (11,650); 226 sh (846), 269 (769). The Mass Spectrum showed a main ion of $M^+246$.

Following procedures known in the art or as described hereinabove the following additional examples of these cis-amino-amide compounds were prepared:

<u>Example 2</u> Cis-3,4-dichloro-N-[2-(1-pyrrolidinyl)cyclopentyl]benzamide, and its 4-methylbenzenesulfonate salt, as its monohydrate

| M.P. | |
|---|---|
| 215°-216°C. (methanol/ether) | Anal. Calcd. for $C_{16}H_{20}Cl_2N_2O \cdot C_7H_8SO_3 \cdot H_2O$<br>% Calcd.: C, 55.08; H, 6.03; N, 5.59; Cl, 14.14; S, 6.40 |

% Found : C, 55.08; H, 5.69; N, 5.63; Cl, 14.34; S, 6.53

<u>Example 3</u> Cis-3,4-dichloro-N-methyl-N-[2-(1-pyrrolidinyl)cyclopentyl]-benzamide, and its monohydrochloride salt

| | |
|---|---|
| <u>M.P.</u> | Anal. Calcd. for $C_{17}H_{22}Cl_2N_2O$ |
| 245°-247°C. | % Calcd.: C, 54.05; H, 6.14; N, 7.42; Cl, 28.16 |
| (methanol/ether) | % Found : C, 54.20; H, 6.08; N, 7.41; Cl, 28.31 |

<u>Example 4</u> Cis-3,4-dichloro-N-[2-(1-pyrrolidinyl)cyclopentyl]benzene-acetamide

| | |
|---|---|
| <u>M.P.</u> | Anal. Calcd. for $C_{17}H_{22}Cl_2N_2O$ |
| 106°C. | % Calcd.: C, 59.82; H. 6.50; N, 8.21; Cl, 20.78 |
| (ether) | % Found : C, 59.94; H, 6.51; N, 8.21; Cl, 20.66 |

<u>Example 5</u> Cis-3,4-dichloro-N-methyl-N-[2-(1-pyrrolidinyl)cyclohexyl]benzeneacetamide

| | |
|---|---|
| <u>M.P.</u> | Anal. Calcd. for $C_{19}H_{2}6Cl_2N_2O$ |
| None (oil) | % Calcd.: C, 61.78; H, 7.10; N, 7.59; Cl, 19.20 |
| | % Found : C, 61.39; H, 7.15; N, 7.55; Cl, 18.51 |

<u>Example 6</u> Cis-3,4-dichloro-N-[2-(1-pyrrolidinyl)cyclohexyl]-benzamide, and its hydrochloride salt

| | |
|---|---|
| <u>M.P.</u> | Anal. Calcd. for $C_{17}H_{22}Cl_2N_2O\cdot HCl$ |
| | HCl |
| 265°-267°C. | % Calcd.: C, 54.05; H, 6.14; N, 7.42; Cl, 28.16 |
| (methanol/ether) | % Found : C, 54.36; H, 6.30; N, 7.41; Cl, 28.16 |

<u>Example 7</u> Cis-3,4-dichloro-N-methyl-N-[2-(1-pyrrolidinyl)-cyclohexyl]benzamide and its monohydrochloride

| | |
|---|---|
| <u>M.P.</u> | Anal. Calcd. for $C_{18}H_{24}Cl_2N_2O\cdot HCl$ |
| 229°C. | |
| (methanol/ether) | % Calcd.: C, 55.18; N, 6.43; N, 7.18; Cl, 27.15 |
| | % Found : C, 55.43; H, 6.50; N, 7.18, Cl, 27.49 |

<u>Example 8</u> Cis-3,4-dichloro-N-[2-(dimethylamino)cyclohexyl]benzeneacetamide

| | |
|---|---|
| <u>M.P.</u> | Anal. Calcd. for $C_{16}H_{22}Cl_2N_2O$ |
| 111°-112°C. | % Calcd.: C, 58.36; H, 6.74; N, 8.51; Cl, 21.54 |
| (ether) | % Found : C, 58.70; H, 6.75; N, 8.51; Cl, 21.39 |

Example 9 Cis-3,4-dichloro-N-[2-(dimethylamino)cyclohexyl]benzamide, and its monohydrochloride

| | |
|---|---|
| M.P. | Anal. Calcd. for $C_{15}H_{20}Cl_2N_2O \cdot HCl$ |
| 231°-232°C. | |
| (methanol/ether) | % Calcd.: C, 51.22; H, 6.02; N, 7.97; Cl, 30.24 |
| | % Found : C, 51.59; H, 6.15; N, 7.84; Cl, 30.09 |

Example 10 Cis-3,4-dichloro-N-methyl-N-[2-(dimethylamino)cyclohexyl]-benzeneacetamide and its p-toluenesulfonate salt

| | |
|---|---|
| M.P. | Anal. Calcd. for $C_{17}H_{24}Cl_2N_2O \cdot C_7H_8SO_3$ |
| 175°-176°C. | % Calcd.: C, 55.91; H, 6.26; N, 5.44; Cl, 13.76; |
| (methanol/ether) | S, 6.22 |
| | % Calcd.: C, 55.89; H, 6.42; N, 5.69; Cl, 13.81, |
| | S, 6.51 |

Example 11 Cis-4-(trifluoromethyl)-N-[2-(dimethylamino)cyclohexyl]benzamide, and its monohydrochloride

| | |
|---|---|
| M.P. | Anal. Calcd. for $C_{16}H_{21}N_2F_3O \cdot HCl$ |
| 183°-184°C. | % Calcd.: C, 54.77; H, 6.32; N, 7.99; F, 16.25; |
| (methanol/ether) | Cl, 10.11 |
| | % Found : C, 54.57; H, 6.43; N, 7.94; Cl, 10.08, |
| | F, 16.37 |

Example 12 Cis-3,4-dichloro-N-[2-(1-pyrrolidinyl)cyclohexyl]benzene-acetamide

| | |
|---|---|
| M.P. | Anal. Calcd. for $C_{18}H_{24}ClN_2O$ |
| 133°-134°C. | % Calcd.: C, 60.79; H. 6,68; N, 7.89; Cl, 19.96 |
| | % Found : C, 60.79; H, 6.68; N. 7.89; Cl, 20.18 |

Example 13 Anticonvulsant Use Test Results -Electroshock Test

This example exemplifies anticonvulsant activity properties of representative compounds of this invention in a standard laboratory animal electroshock test.

Male CF-1 mice were injected subcutaneously with the test compound, and after the compound was absorbed, the test mice were subjected to maximal transpinnal electroshock (10mA, 0.2 sec.). Protection from Central Nervous System (CNS) seizure by the test compound was judged by the absence of the tonic extensor convulsion. Each test drug substance or compound was formulated into 0.25 percent w/v aqueous methylcellulose solution and administered at a volume dose of 10 ml. per kilogram of body weight. A group of six mice was treated and tested at each dose level, and decreasing doses (0.3 log intervals) were administered to such groups of mice until response activity was low enough so that $ED_{50}$ dose calculations within 95 percent confidence intervals could be established. Most of these exemplified compounds lack any significant analgesic activity ($ED_{50}$ greater than 50 in standard HCl writing analgesic test).

TABLE 1

ELECTROSHOCK TEST RESULTS

($ED_{50}$, subcutaneous route, mg./kg.) (95 percent, Confidence Index Range)

| Example No. Compound | Electroshock ($ED_{50}$, mg./kg.) |
|---|---|
| 3 | 32 |
| 4 | 50 |
| 6 | 18 |
| 7 | 13 (9-17) |
| 8 | 50 (31-81) |
| 9 | 31 |
| 10 | 40 |
| 11 | 25 (17-30) |
| 12 | 62 |

Example 14

One thousand tablets for oral use, each containing 175 mg. of cis-3,4-dichloro-N-methyl-N-[2-(1-pyrrolidinyl)cyclohexyl]benzamide hydrochloride as the essential active ingredient are prepared from the following ingredients:

Essential active ingredient: 175 gm.
Methylcellulose, U.S.P (15 cps.) -6.5 gm.
Talc -20 gm.
Calcium Stearate -2.0 gm.

The essential active ingredient and dicalcium phosphate are mixed well, granulated with 7.5% aqueous solution of methylcellulose, passed through a No. 8 screen and dried carefully. The dried granules are passed through No. 12 screen, mixed with the talc and stearate and com pressed into tablets. These tablets are useful in the treatment of adult humans at a dose of 1 tablet to 4 times a day as needed to prevent convulsions.

Example 15

One thousand two-piece hard gelatin capsules for oral use, each capsule containing 100 mgs. of cis-3,4-dichloro-N-methyl-N-[2-(1-pyrrolidinyl)cyclohexyl]benzamide hydrochloride as the essential active ingredient are prepared from the following ingredients:

Essential active ingredient -100 gm.
Lactose, U.S.P. -100 gm.
Starch, U.S.P. -10 gm.
Talc, U.S.P. -5 gm.
Calcium Stearate -1 gm.

The finely powdered materials are mixed thoroughly, then filled into hard gelatin capsules of appropriate size.

One capsule four times a day is useful for the treatment of adult humans to block the occurrence of CNS convulsive seizures.

Example 16

A sterile aqueous suspension suitable for intramuscular injection and containing in each milliliter 50 mg. of the Example 16 essential active ingredient is prepared from the following ingredients:

Essential active ingredient -5 gm.
Polyethylene glycol 4000, U.S.P. -3 gm.
Sodium chloride -0.9 gm.
Polysorbate 80, U.S.P. -0.4 gm.
Sodium metabisulfite -0.1 gm.
Methylparaben, U.S.P. -0.18 gm.
Propylparaben, U.S.P. -0.02 gm.
Water for injection, q.s. to -100 ml.

The preceding sterile injectable is useful in the treatment of CNS convulsions in adult humans at a dose of 1/2 to 2 ml.

GENERAL FORMULAS

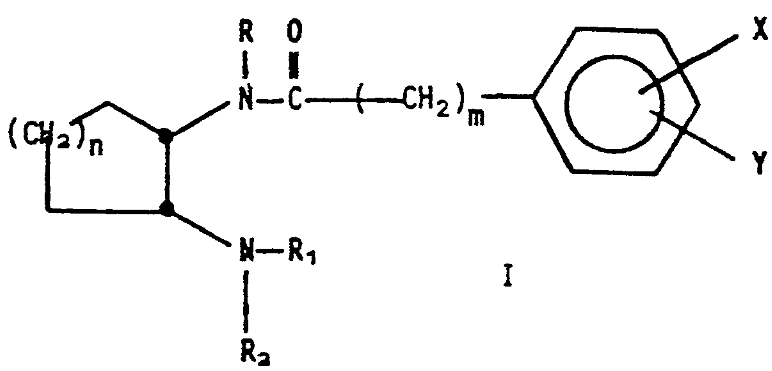

I

SCHEME I

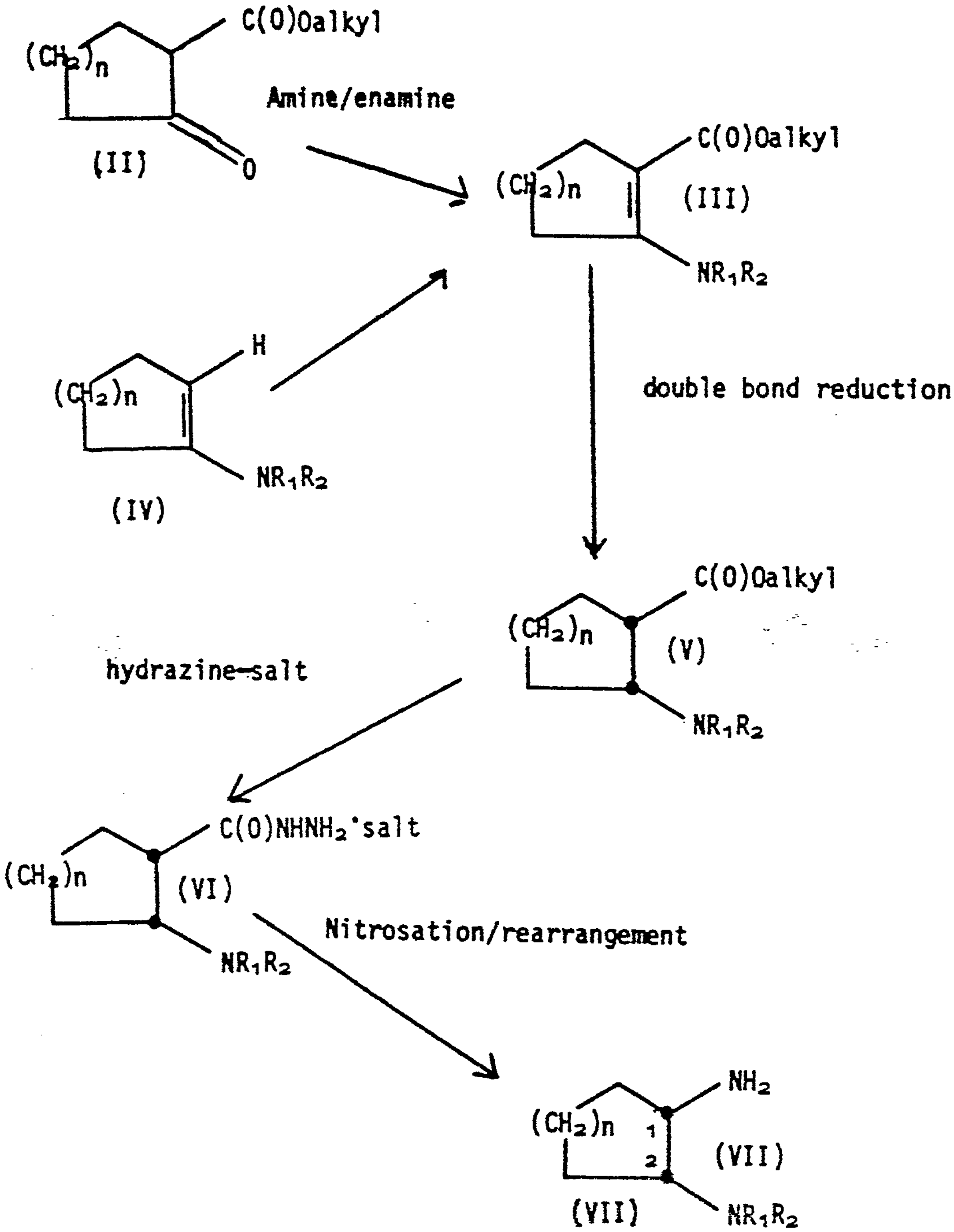


(II) (III) (IV) (V) (VI) (VII)

SCHEME I - CONTINUED

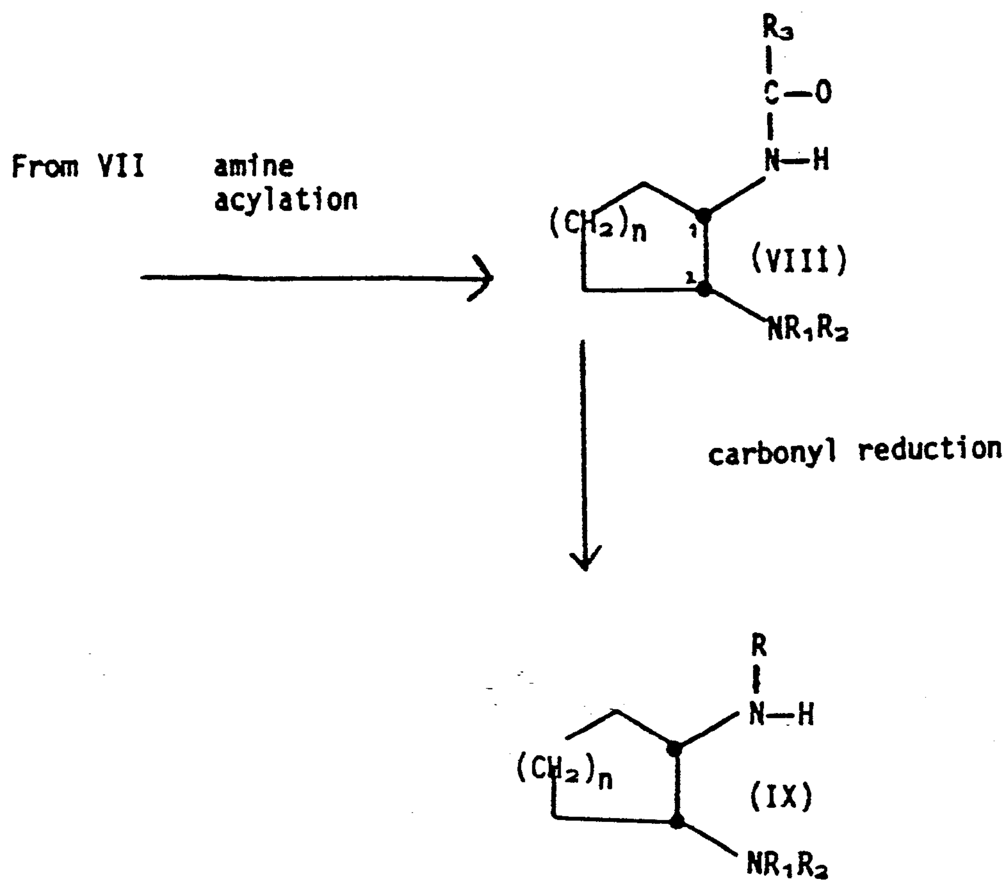


(VIII)

(IX)

SCHEME II

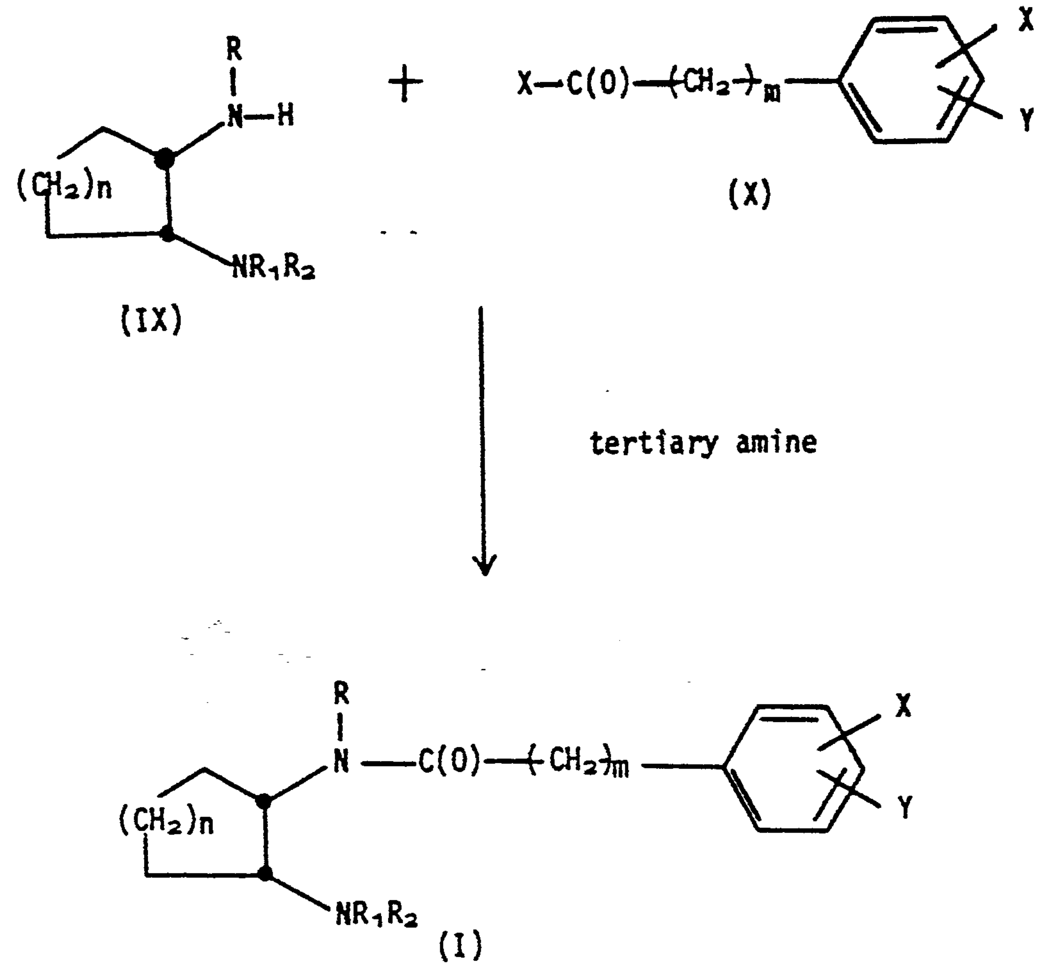


## Claims

1. A method for preventing or treating Central Nervous System seizures in a warm-blooded animal patient which comprises administering to such patient suffering from seizures or likely to experience seizures an amount of a compound of the formula

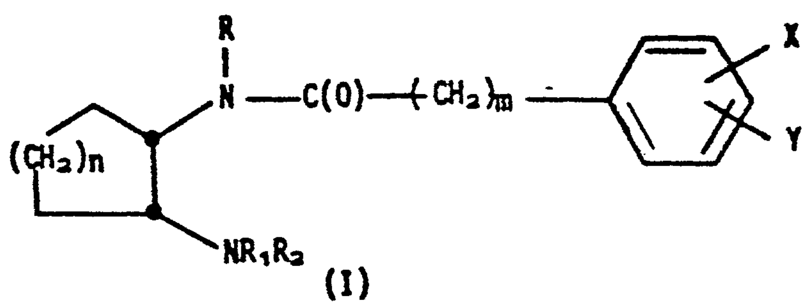

(I)

(I)

where the dotted line bonds between the ring and the two nitrogens denote the cis orientation,
n is 1 or 2;

m is 0 or 1;
R is hydrogen or $C_1$ to $C_3$-alkyl,
$R_1$ and $R_2$ taken separately, are each independently or $C_1$ to $C_3$-alkyl,
$R_1$ and $R_2$, taken together with the nitrogen to which they are bonded complete a pyrrolidine ring,
X and Y are each selected from the group consisting of hydrogen and a halogen having an atomic number of from 9 to 35, or X is hydrogen and Y is trifluoromethyl,
or a pharmaceutically acceptable salt thereof, which amount is effective to prevent or reduce the effects of such CNS seizure disorder in said patient.

2. A method as defined in Claim 1 wherein the CNS seizure preventing or treating compound I is a compound selected from the group consisting of
(a) cis-N-[2-(dimethylamino)cyclohexyl]benzamide,
(b) cis-3,4-dichloro-N-methyl-N-[2-(1-pyrrolidinyl)cyclopentyl]benzamide,
(c) cis-3,4-dichloro-N-methyl-N-[2-(1-pyrrolidinyl)cyclohexyl]benzeneacetamide,
(d) cis-3,4-dichloro-N-[2-(1-pyrrolidinyl)cyclohexyl]benzamide,
(e) cis-3,4-dichloro-N-methyl-N-[2-(1-pyrrolidinyl)cyclohexyl]benzamide,
(f) cis-3,4-dichloro-N-[2-(dimethylamino)cyclohexyl]benzeneacetamide,
(g) cis-3,4-dichloro-N-[2-(dimethylamino)cyclohexyl]benzamide,
(h) cis-4-trifluoromethyl-N-[2-(dimethylamino)cyclohexyl]benzamide, and
(i) cis-3,4-dichloro-N-[2-(1-pyrrolidinyl)cyclohexyl]benzeneacetamide,
or a pharmacologically acceptable salt of such a compound.

3. A method as defined in Claim 2 wherein the CNS seizure-preventing or treating compound administered to said patient is a compound selected from the group consisting of cis-3,4-dichloro-N-methyl-N-[2-(1-pyrrolidinyl)cyclohexyl]benzamide, and
cis-3,4-dichloro-N-[2-(1-pyrrolidinyl)cyclohexyl]benzamide, or a pharmaceutically acceptable salt thereof.

4. A method as defined in Claim 3 wherein the CNS seizure-preventing or treating compound is cis-3,4-dichloro-N-[2-(1-pyrrolidinyl)cyclohexyl-pyrrolidinyl)cyclohexyl]benzamide hydrochloride.

5. A method as defined in Claim 3 wherein the CNS seizure-preventing or blocking compound is cis-3,4-dichloro-N-methyl-N-[2-1(pyrrolidinyl)cyclohexyl]benzamide hydrochloride.

6. A compound selected from the group consisting of
cis-3,4-di-chloro-N-[2-(1-pyrrolidinyl)cyclopentyl]benzeneacetamide,
cis-3,4-dichloro-N-methyl-N-[2-(1-pyrrolidinyl)cyclopenpentyl]benzamide,
cis-3,4-dichloro-N-[2-(1-pyrrolidinyl)cyclohexyl]benzamide and
cis-3,4-dichloro-N-methyl-N-[2-(1-pyrrolidinyl)cyclohexyl]benzamide, or a pharmaceutically acceptable salt thereof.

7. A compound according to Claim 6 which is cis-3,4-dichloro-N-[2-(1-pyrrolidinyl)cyclopentyl]-benzeneacetamide, or a pharmaceutically acceptable salt thereof.

8. A compound according to Claim 6 which is cis-3,4-dichloro-N-methyl-N-[2-(1-pyrrolidinyl)-cyclopentyl]benzamide, or a pharmaceutically acceptable salt thereof.

9. A compound according to Claim 6 which is cis-3,4-dichloro-N-[2-(1-pyrrolidinyl)cyclohexyl]-benzamide, or a pharmaceutically acceptable salt thereof.

10. A compound according to Claim 6 which is cis-3,4-dichloro-N-methyl-N-[2-(1-pyrrolidinyl)-cyclohexyl]benzamide, or a pharmaceutically acceptable salt thereof.

11. A composition for preventing or treating Central Nervous System seizures in a warm-blooded animal patient which comprises a compound of the formula

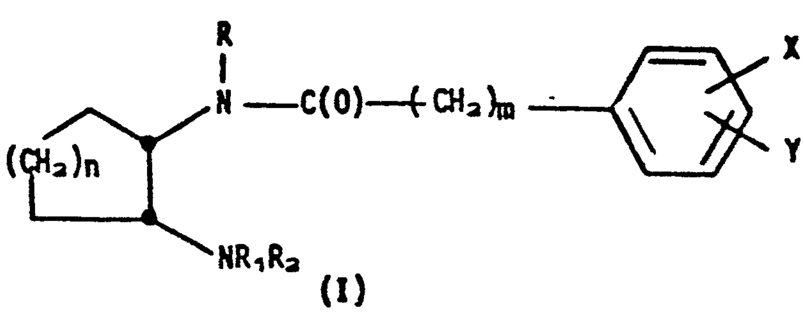

(I)

(I)

where the dotted line bonds between the ring and the two nitrogens denotes the cis orientation,

n is 1 or 2;
m is 0 or 1;
R is hydrogen or $C_1$ to $C_3$-alkyl,
$R_1$ and $R_2$ taken separately, are each independently or $C_1$ to $C_3$-alkyl,
$R_1$ and $R_2$, taken together with the nitrogen to which they are bonded complete a pyrrolidine ring,
X and Y are each selected from the group consisting of hydrogen and a halogen having an atomic number of from 9 to 35, or X is hydrogen and Y is trifluoromethyl,
or a pharmaceutically acceptable salt thereof as an active ingredient.

12. A composition as defined in Claim 11 wherein the CNS seizure preventing or treating compound I is a compound selected from the group consisting of
(a) cis-N-[2-(dimethylamino)cyclohexyl]benzamide,
(b) cis-3,4-dichloro-N-methyl-N-[2-(1-pyrrolidinyl)cyclopentyl]benzamide,
(c) cis-3,4-dichloro-N-methyl-N-[2-(1-pyrrolidinyl)cyclohexyl]benzeneacetamide,
(d) cis-3,4-dichloro-N-[2-(1-pyrrolidinyl)cyclohexyl]benzamide,
(e) cis-3,4-dichloro-N-methyl-N-[2-(1-pyrrolidinyl)cyclohexyl]benzamide
(f) cis-3,4-dichloro-N-[2-(dimethylamino)cyclohexyl]benzeneacetamide,
(g) cis-3,4-dichloro-N-[2-(dimethylamino)cyclohexyl]benzamide,
(h) cis-4-trifluoromethyl-N-[2-(dimethylamino)cyclohexyl]benzamide, and
(i) cis-3,4-dichloro-N-[2-(1-pyrrolidinyl)cyclohexyl]benzeneacetamide,
or a pharmacologically acceptable salt of such a compound.

13. A composition as defined in Claim 12 wherein the CNS seizure-preventing or treating compound administered to said patient is a compound selected from the group consisting of cis-3,4-dichloro-N-methyl-N-[2-(1-pyrrolidinyl)cyclohexyl]benzamide, and
cis-3,4-dichloro-N-[2-(1-pyrrolidinyl)cyclohexyl]benzamide, or a pharmaceutically acceptable salt thereof.

14. A composition as defined in Claim 13 wherein the CNS seizure-preventing or treating compound is cis-3,4-dichloro-N-[2-(1-pyrrolidinyl)cyclohexyl]benzamide hydrochloride.

15. A composition as defined in Claim 13 wherein the CNS seizure-preventing or blocking compound is cis-3,4-dichloro-N-methyl-N-[2-(1-pyrrolidinyl)cyclohexyl]benzamide hydrochloride.

European Patent Office

# EUROPEAN SEARCH REPORT

Application number

EP 86 30 8174

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | FR-A-2 370 722 (UPJOHN COMPANY) * Claims * --- | 11-15 | A 61 K 31/165<br>A 61 K 31/40<br>C 07 D 295/12 |
| D,X | FR-A-2 370 723 (UPJOHN COMPANY) * Claims; example 32 * --- | 6-15 | |
| A | US-A-4 463 013 (R.J. COLLINS et al.) * Claims * --- | 11-15 | |
| P,X | CHEMICAL ABSTRACTS, vol. 104, no. 25, 23rd June 1986, abstract no. 219015h, Columbus, Ohio, US; F.C. TORTELLA et al.: "U50,488, a highly selective kappa opioid: anticonvulsant profile in rats", & J. PHARMACOL. EXP. THER. 1986, 237(1), 49-53 ----- | 11-15 | |
| | | | **TECHNICAL FIELDS SEARCHED (Int. Cl.4)** |
| | | | A 61 K 31/00<br>C 07 D 295/00 |

The present search report has been drawn up for all claims

| Place of search | Date of completion of the search | Examiner |
|---|---|---|
| THE HAGUE | 02-02-1987 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document